# EUROPÄISCHE PATENTSCHRIFT

(11) **EP 1 800 648 B1**
(45) Veröffentlichungstag und Bekanntmachung des Hinweises auf die Patenterteilung: **28.11.2012**
(21) Anmeldenummer: 06125764.8
(22) Anmeldetag: 11.12.2006
(51) Int. Cl.: A61K 8/06, A61K 8/34, A61K 8/37, A61K 8/86, A61Q 5/06

(54) **Mikroemulsion zum Stylen der Haare**
Hair styling composition in the form of a microemulsion
Composition de mise en forme des cheveux sous forme de microémulsion

(30) Priorität: 13.12.2005 DE 102005059835
(43) Veröffentlichungstag der Anmeldung: 27.06.2007
(73) Patentinhaber: Beiersdorf AG, 20253 Hamburg (DE)
(72) Erfinder: Detert, Marion, 22455 Hamburg (DE); Pilzner, Anke, 22459, Hamburg (DE)

(56) Entgegenhaltungen:
- EP-A- 1 386 601
- EP-A- 1 559 395
- WO-A-2004/112731
- JP-A- 2003 012 477
- US-A1- 3 458 624

## Beschreibung

Die vorliegende Erfindung betrifft neuartige Haarstylingmittel in Form einer Mikroemulsion. Der Wunsch, schön und attraktiv auszusehen, ist von Natur aus im Menschen verwurzelt. Auch wenn das Schönheitsideal im Laufe der Zeit Wandlungen erfahren hat, so ist das Streben nach einem makellosen Äußeren immer das Ziel der Menschen gewesen. Einen wesentlichen Anteil an einem schönen und attraktiven Äußeren hat dabei das Aussehen der Haare. Das Haar wird gerne frisiert, also in Form gebracht. Diese gestaltende Tätigkeit wird auch als "Stylen" bezeichnet.

Der ganze menschliche Körper mit Ausnahme der Lippen, der Handinnenflächen und der Fußsohlen ist behaart, zum Großteil allerdings mit kaum sichtbaren Wollhärchen. Wegen der vielen Nervenden an der Haarwurzel reagieren Haare empfindlich auf äußere Einflüsse wie Wind oder Berührung und sind daher ein nicht zu unterschätzender Bestandteil des Tastsinns. Die wichtigste Funktion des menschlichen Kopfhaares dürfte allerdings heute darin bestehen, das Aussehen des Menschen in charakteristischer Weise mitzugestalten. Ähnlich wie die Haut erfüllt es eine soziale Funktion, da es über sein Erscheinungsbild erheblich zum Selbstwertgefühl des Individuums beiträgt.

Das Haar besteht aus dem frei aus der Haut herausragenden Haarschaft - dem keratinisierten (toten) Teil, der das eigentlich sichtbare Haar darstellt - und der in der Haut steckenden Haarwurzel - dem lebenden Teil, in dem das sichtbare Haar ständig neu gebildet wird. Der Haarschaft seinerseits ist aus drei Schichten aufgebaut: einem zentralen Teil - dem so genannten Haarmark (Medulla), welches allerdings beim Menschen zurückgebildet ist und oft gänzlich fehlt - ferner dem Mark (Cortex) und der äußeren, bis zu zehn Lagen starken Schuppenschicht (Cuticula), die das ganze Haar umhüllt.

Natürliches Haar hängt meist schlaff und ohne Volumen vom Kopf herab. Ein Ziel der Haarpflege ist es daher, dem Haar Fülle und Frisur zu geben. Zur temporären Verformung des Haares und Gestaltung (engl. Styling) vielseitiger Frisuren verwendet man verschiedene Haarstylingmittel, darunter auch Wachse.

Wachse sind bekannte Produkte zum Stylen der Haare, also zur temporären Verformung und Stabilisierung der Frisur. Vorteil solcher Produkte ist, dass sich die Frisur stylen lässt, ohne dass das Haar verklebt oder zu fest oder hart wird (wie bei einem Gel). Nachteil dieser Produkte ist jedoch, dass der Griff des Haares oft als zu fettig oder ölig empfunden wird und dass das Haar beschwert wird und somit die erstellte Frisur nicht in der gewünschten Form bleibt.

Zwar kennt der Stand der Technik mit EP 490053 ein Haarkurmittel in Form einer Mikroemulsion, solche Produkte konnten die Nachteile des Standes der Technik jedoch nicht beseitigen. Die in EP 915693 beschriebene O/W-Emulsion zur Reinigung der Haut eignet sich ebenfalls nicht befriedigend zur Gestaltung von Frisuren. DE 19509079 offenbart transparente Mikroemulsionen vom Typ Öl-in-Wasser, die sich ebenfalls unter anderem aufgrund ihrer Dünnflüssigkeit nicht eignen, um eine Frisur zu gestalten. EP930866 schließlich offenbart Gele auf der Basis von Mikroemulsionen. Diese sind unter anderem aufgrund ihrer Fliesseigenschaften nicht geeignet, den Nachteilen des Standes der Technik abzuhelfen.

Andere Ansätze zur Beseitigung der Nachteile des Standes der Technik sind nicht bekannt. Zwar beschreiben EP 1302193 und EP 1559402 Haarwachse. Beide Dokumente schweigen aber hinsichtlich einer Verminderung der wachsigen Haptik des Haarwachses.

Harstylingprodukte nach EP 559395 A1 weisen eine Emulgatorkombination aus einem Emulgator A aus Gruppe der Monoester, Diester, Polyester von Polyolen mit HLB-Wert von 12 bis 15, b) Emulgator B aus der Gruppe der polyethoxylierten Emulgatoren mit einem HLB-Wert von 12 - 17 auf, deren Viskosität von flüssig bis wachsartig fest reicht.

Es wurde nun überraschend eine kosmetische Zubereitung zur Frisurgestaltung in Form einer festen O/W-Mikroemulsion enthaltend
- mindestens einen ersten Emulgator A,
- mindestens einen zweiten Emulgator B,
- mindestens einen mehrwertigen Alkohol C,
wobei der Emulgator A aus der Gruppe der Monoester, Diester, Polyester von Polyolen mit einem HLB-Wert von 2 bis 6,
der Emulgator B aus der Gruppe der polyethoxylierten Emulgatoren mit einem HLB Wert von 12 bis 17 gewählt wird und dass die Konzentration von Emulgator A und Emulgator B zusammengenommen größer als 21 Gew.% ist, , dadurch gekennzeichnet, dass ein weiterer mehrwertiger Alkohol D enthalten ist, wobei es sich um ein C4 Alkandiol handelt, gefunden. Diese stellt eine feste Mikroemulsion dar, die verglichen mit einem Styling Wachs die gleichen Vorteile zum Stylen der Haare besitzt, sich aber überraschenderweise deutlich in den sensorischen Eigenschaften unterscheidet: das Haar wird weder zu fettig noch wird es unangenehm beschwert. Die positiven taktilen Unterschiede werden vom Verbraucher in Blindversuchen deutlich wahrgenommen.

Dabei ist es bevorzugt, wenn das Massenverhältnis aller Emulgatoren (A und B) zu dem oder den mehrwertigen Alkohol/en zwischen 2:1 und 20:1 liegt, besonders bevorzugt wird ein Verhältnis von 3,5:1 bis 5:1.

Dabei ist es bevorzugt, wenn Emulgator A Ceteareth-20 darstellt. Weiter ist bevorzugt, wenn Emulgator B Glycerylisostearat darstellt. Weiter ist bevorzugt, wenn der mehrwertige Alkohol C Glycerin ist.

Besonders bevorzugt ist es, wenn der mehrwertige Alkohol D Methylpropandiol ist. Besonders bevorzugt ist es, wenn der relative wachsige Haargriffwert der Zubereitung oder der verwendungsgemäßen Zubereitung 2 bis 10 beträgt. Besonders bevorzugt ist es, wenn die Konzentration von Alkohol C und Alkohol D zusammengenommen kleiner als 10 Gew.-% ist. Besonders bevorzugt ist es, wenn die erfindungsgemäße Zubereitung oder Verwendung eine Transmission von 80 - 90% gemessen mit UV/VIS Spektrometer vom Typ HP 8453 bei eine Wellenlänge von 420 nm hat.

Der spezielle Griff der Haare wurde von einem geschulten Panel untersucht und als besonders vorteilhaft gegenüber herkömmlichen Wachsen evaluiert. Die Bewertung der für die jeweilige Produktkategorie relevanten, sensorischen Eigenschaften erfolgte durch ein Expertenpanel.

Für Stylingprodukte ist die Klebrigkeit die Hauptzielvariable und der Griff und die Kämmbarkeit werden als Nebenzielvariablen bewertet. Dazu beurteilen Experten produktrelevante sensorische Eigenschaften im Paarvergleich mit einer Referenz an europäischen Naturhaarsträhnen. Die sensorische Beurteilung erfolgt immer im Vergleich zu einer Referenz. Die Referenz wird für jede Studie definiert. In der Regel werden drei Paarvergleiche durchgeführt, wobei sowohl der Vergleich von drei Produkten gegenüber einer Referenz wie auch der Vergleich von 2 Produkten gegen eine Referenz und gegeneinander möglich sind. Es erfolgen Bewertungen im nassen und im trockenen Haar an geschädigten oder ungeschädigten Echthaarsträhnen. Sensorische Bewertungen nutzen die menschliche Sensibilität gegenüber Reizen. In diesem Fall werden mit Hilfe des Tastsinns geschulter Testpersonen die taktilen Sinneseindrücke zur Produktbeurteilung genutzt. Die variierende Sensibilität des Menschen für bestimmte Sinneseindrücke kann trainiert und durch den Ausbau neuronaler Netzwerke verbessert werden.

Für eine Testreihe werden mindestens 12 Testpersonen benötigt. Als Testpersonen bzw. Experten gelten dabei Personen, die in der Bewertung von sensorischen Eigenschaften an Haaren durch eine Probephase und durch den ständigen Umgang trainiert sind. Für die Beurteilungen stehen eine ausreichende Anzahl an Experten zur Verfügung, die je nach Verfügbarkeit für die jeweilige Studie ausgewählt werden.

Für jeden Produktvergleich werden zwei Strähnen benötigt. Jeder Paarvergleich wird dann je nach Trennschärfe durch mindestens 12 Experten beurteilt.

Als deskriptive Auswertung erfolgt die Angabe der relativen und absoluten Häufigkeiten der Beurteilungen, unterteilt in die Kategorien "zu Gunsten der Referenz", "gleich" und "zu Gunsten des Produktes", so wie die grafische Darstellung der Ergebnisse in Form von Histogrammen.

Die induktive Auswertung der Paarvergleiche erfolgt mit dem Vorzeichentest nach Dixon und Mood.

Von jeder Studie wird ein Abschlussbericht erstellt. In dem Bericht sind in jedem Fall die folgenden Angaben zu dokumentieren: Testprodukte und Codierungen, verwendeter Haartyp, Chargenbezeichnung der verwendeten Haartressen, Messklima, Art und Durchführung der Produktapplikation, Messparameter der Universalprüfmaschine.

Literatur: Sensory Evaluation Techniques, 3rd Edition, M. Meilgaard, G. V. Civille, B.T. Carr, CRC Press New York

### Beispiele Gloss-Wachs

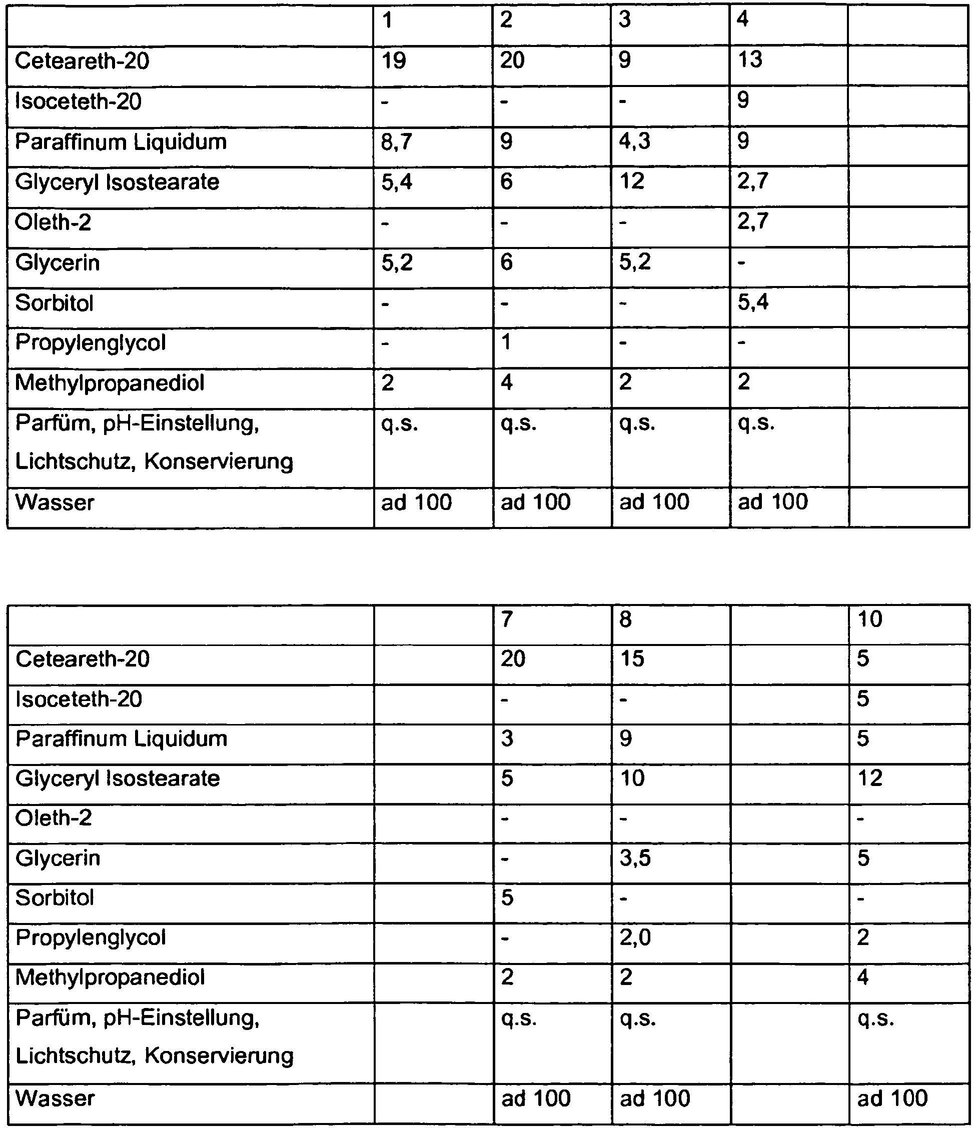

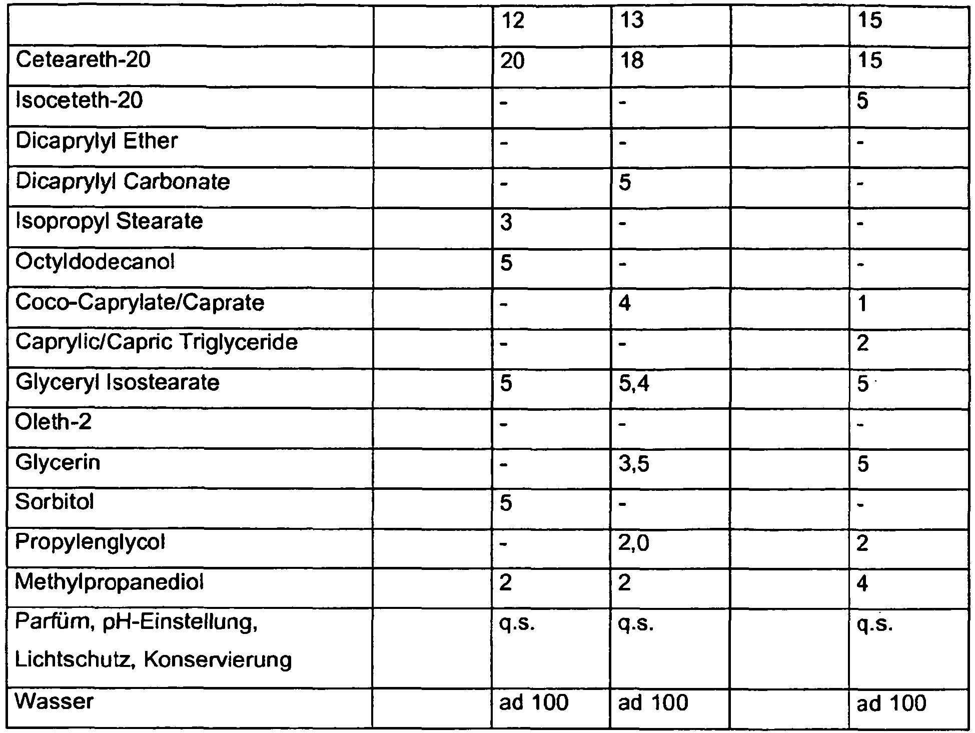

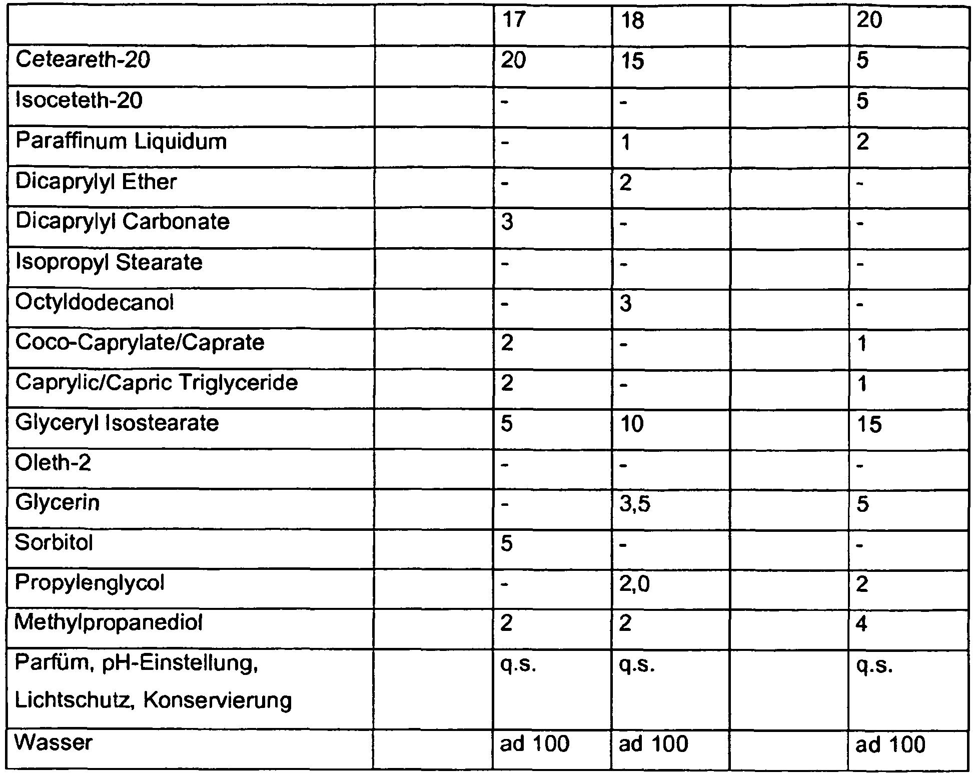

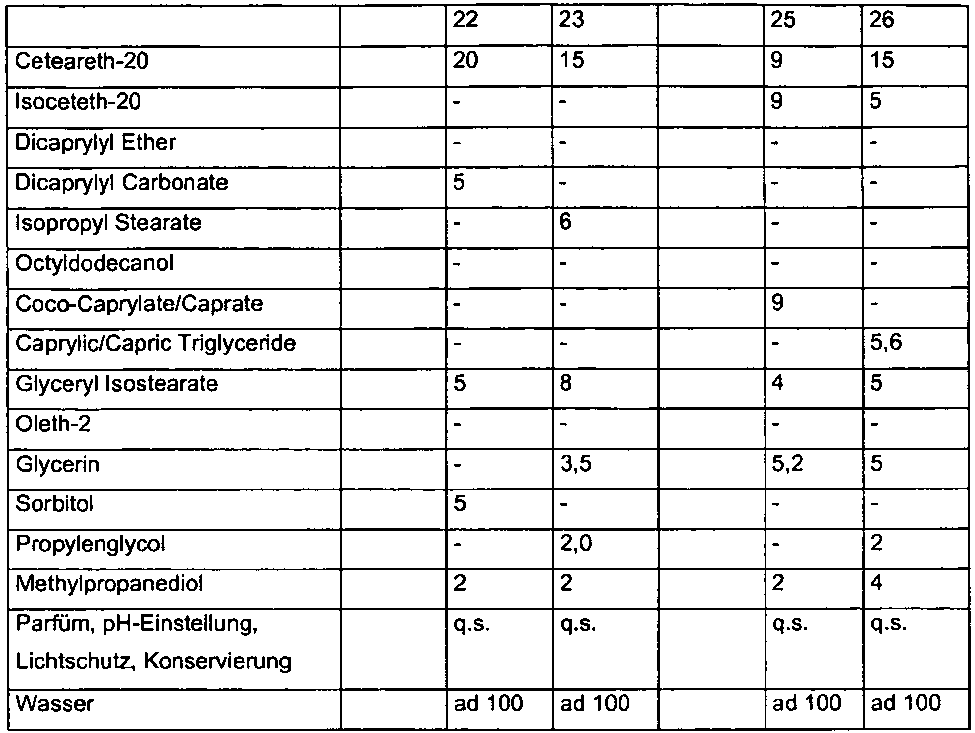

Sensorische Bewertung von mit verschiedenen Wachsen vorbehandelten ungeschädigten Haarproben im Paneltest auf Verteilen, Griff und Glanz (SBH.029), 18.08.2005

| | |
|---|---|
| 19807-10 | Erfindung gemäß Bsp. 2 |
| 19807-20 | NHC Styling Silky Shine Wax-RL 2005 |

Sensorische Bewertung an Haarsträhnen, Paarvergleich;
Haarmaterial: EN-Haar, ungeschädigt.

Ziel der Untersuchung war die Evaluierung der Produktverteilung zwischen den Fingern und der Produktverteilung im Haar, des Griffes am Haar und des Haarglanzes von mit verschiedenen Haarwachsen vorbehandelten Haarproben bei praxisnaher Anwendung und Bewertung durch ein Experten-Panel.
Haarmaterial: EN-Haar ungeschädigt.
Die Bewertungen wurden im trockenen Haar durchgeführt.
Eine definierte Applikationsmenge wurde von den Experten selbst in die Haartresse eingearbeitet.

Es wurde ein Vergleich zur Referenz gemacht (Paarvergleich).

| | N | (+) | (=) | (-) | Dixon & Mood |
|---|---|---|---|---|---|
| 20 vs. 10 | | 20 besser | kein Unterschied | 10 besser | Ergebnis |
| Verteilen_Finger | 18 (100%) | 13 (72,2%) | 1 (5,6%) | 4 (22,2%) | n.s. |
| Verteilen_Haar | 18 (100%) | 10 (55,6%) | 3 (16,7%) | 5 (27,8%) | n.s. |
| klebrig | 18 (100%) | 4 (22,2%) | 3 (16,7%) | 11 (61,1%) | n.s. |
| fettig | 18 (100%) | 2 (11,1%) | 1 (5,6%) | 15 (83,3%) | + |
| wachsig | 18 (100%) | 3 (16,7%) | 3 (16,7%) | 12 (66,7%) | + |
| geschmeidig | 18 (100%) | 5 (27,8%) | 2 (11,1%) | 11 (61,1%) | n.s. |
| GLANZ | 18 (100%) | 9 (50,0%) | 5 (27,8%) | 4 (22,2%) | n.s. |

Der relative wachsige Haargriffwert berechnet sich aus den dargestellten Ergebnissen durch Bezug auf den Standard "NHC Styling Silky Shine WAX-RL2005" nach der Formel: Antworten 10 besser / Antworten 20 besser für den Wert "wachsig".
In diesem Fall ergibt sich ein relativer wachsiger Haargriffwert von 12/3 = 4.

### Vergleich NHC Styling Silky Shine WAX-RL2005 (20) gegen Referenz Erfindung gemäß Beispiel2. (10):

Bei der Behandlung mit NHC Styling Silky Shine WAX-RL2005 (20) konnte bezüglich der Eigenschaften "Verteilen_Finger", "Verteilen_Haar", "klebrig", "geschmeidig" und "Glanz" gegenüber der Referenz NHC Styling Gloss Produkt GLOSSWO06:66 (10) kein statistisch signifikanter Unterschied festgestellt werden. Bezüglich der Eigenschaften "fettig" und "wachsig" konnte eine signifikante Überlegenheit der Referenz NHC Styling Gloss Produkt GLOSSWO06:66 (10) gegenüber NHC Styling Silky Shine WAX-RL2005 (20) nachgewiesen werden.

## Patentansprüche

1. Kosmetische Zubereitung zur Frisurgestaltung in Form einer festen O/W-Mikroemulsion enthaltend
- mindestens einen ersten Emulgator A,
- mindestens einen zweiten Emulgator B,
- mindestens einen mehrwertigen Alkohol C,
wobei der Emulgator A aus der Gruppe der Monoester, Diester, Polyester von Polyolen mit einem HLB-Wert von 2 bis 6,
der Emulgator B aus der Gruppe der polyethoxylierten Emulgatoren mit einem HLB Wert von 12 bis 17 gewählt wird und dass die Konzentration von Emulgator A und Emulgator B zusammengenommen größer als 21 Gew.-% ist, **dadurch gekennzeichnet, dass** ein weiterer mehrwertiger Alkohol D enthalten ist, wobei es sich um ein C4 Alkandiol handelt.

2. Zubereitung nach einem der vorangehenden Patentansprüche **dadurch gekennzeichnet, dass** das Massenverhältnis aller Emulgatoren (A und B) zu dem oder den mehrwertigen Alkohol/en zwischen 2:1 und 20:1 liegt.

3. Zubereitung nach einem der vorangehenden Patentansprüche **dadurch gekennzeichnet, dass** Emulgator A Glycerylisostearat darstellt.

4. Zubereitung nach einem der vorangehenden Patentansprüche **dadurch gekennzeichnet, dass** Emulgator B Ceteareth-20 darstellt.

5. Zubereitung nach einem der vorangehenden Patentansprüche **dadurch gekennzeichnet, dass** der mehrwertige Alkohol C Glycerin ist.

6. Zubereitung nach einem der vorangehenden Patentansprüche **dadurch gekennzeichnet, dass** der mehrwertige Alkohol D Methylpropandiol ist.

7. Zubereitung nach einem der vorangehenden Patentansprüche **dadurch gekennzeichnet, dass** die Konzentration von Alkohol C und Alkohol D zusammengenommen kleiner als 10 Gew.-% ist.

8. Zubereitung nach einem der vorangehenden Patentansprüche **dadurch gekennzeichnet, dass** sie eine Transmission von 80 - 90% gemessen mit UV/VIS Spektrometer vom Typ HP 8453 bei einer Wellenlänge von 420 nm hat.

## Claims

1. Cosmetic preparation for hairstyling in the form of a solid O/W microemulsion comprising
- at least one first emulsifier A,
- at least one second emulsifier B,
- at least one polyhydric alcohol C,
where the emulsifier A is selected from the group of the monoesters, diesters, polyesters of polyols with an HLB value of 2 to 6,
the emulsifier B is selected from the group of the polyethoxylated emulsifiers with an HLB value of 12 to 17 and the concentration of emulsifier A and emulsifier B taken together is greater than 21% by weight, **characterized in that** a further monohydric alcohol D is present which is a C4 alkanediol.

2. Preparation according to Claim 1, **characterized in that** the mass ratio of all emulsifiers (A and B) to the polyhydric alcohol (s) is between 2:1 and 20:1.

3. Preparation according to one of the preceding patent claims, **characterized in that** emulsifier A is glyceryl isostearate.

4. Preparation according to one of the preceding patent claims, **characterized in that** emulsifier B is ceteareth-20.

5. Preparation according to one of the preceding patent claims, **characterized in that** the polyhydric alcohol C is glycerol.

6. Preparation according to one of the preceding patent claims, **characterized in that** the polyhydric alcohol D is methylpropanediol.

7. Preparation according to one of the preceding patent claims, **characterized in that** the concentration of alcohol C and alcohol D taken together is less than 10% by weight.

8. Preparation according to one of the preceding patent claims, **characterized in that** it has a transmission of 80-90% measured with UV/VIS spectrometer model HP 8453 at a wavelength of 420 nm.

## Revendications

1. Préparation cosmétique pour le coiffage, sous forme d'une microémulsion H/E solide contenant
- au moins un premier émulsifiant A,
- au moins un deuxième émulsifiant B,
- au moins un alcool polyhydrique C,
l'émulsifiant A étant choisi dans le groupe des monoesters, diesters, polyesters de polyols ayant une valeur HLB de 2 à 6,
l'émulsifiant B étant choisi dans le groupe des émulsifiants polyéthoxylés ayant une valeur HLB de 12 à 17 et la concentration de l'émulsifiant A et de l'émulsifiant B pris ensemble étant supérieure à 21 % en poids, **caractérisée en ce qu'**est contenu un autre alcool polyhydrique D, consistant en un alcanol en C₄.

2. Préparation selon la revendication 1, **caractérisée en ce que** le rapport massique de tous les émulsifiants (A et B) à l'alcool ou aux alcools polyhydrique(s) est compris entre 2:1 et 20:1.

3. Préparation selon l'une quelconque des revendications précédentes, **caractérisée en ce que** l'émulsifiant A représente l'isostéarate de glycéryle.

4. Préparation selon l'une quelconque des revendications précédentes, **caractérisée en ce que** l'émulsifiant B représente le ceteareth-20.

5. Préparation selon l'une quelconque des revendications précédentes, **caractérisée en ce que** l'alcool polyhydrique C est le glycérol.

6. Préparation selon l'une quelconque des revendications précédentes, **caractérisée en ce que** l'alcool polyhydrique D est le méthylpropanediol.

7. Préparation selon l'une quelconque des revendications précédentes, **caractérisée en ce que** la concentration de l'alcool C et de l'alcool D pris ensemble est inférieure à 10 % en poids.

8. Préparation selon l'une quelconque des revendications précédentes, **caractérisée en ce qu'**elle a une transmission de 80 - 90 %, mesurée avec un spectromètre UV/VIS du type HP 8453 à une longueur d'onde de 420 nm.
